# EUROPEAN PATENT SPECIFICATION

(11) **EP 1 121 948 B1**
(45) Date of publication and mention of the grant of the patent: **23.06.2004**
(21) Application number: 99970029.7
(22) Date of filing: 07.10.1999
(51) Int. Cl.: A61M 1/28

(54) **Device and methods for perfusing peritoneal dialysing fluid**
Vorrichtung und Verfahren zur Perfusion peritonealer Dyalyseflüssigkeit
Dispositif et méthode de perfusion de fluide de dialyse péritonéale

(30) Priority: 07.10.1998 JP 28502998
(43) Date of publication of application: 08.08.2001
(73) Proprietor: Sakai, Asahi, Sakura-shi, Chiba 285-0067 (JP)
(72) Inventor: Sakai, Asahi, Sakura-shi, Chiba 285-0067 (JP)
(74) Representative: Senior, Alan Murray
(86) International application number: PCT/JP1999/005535
(87) International publication number: WO 2000/020052

(56) References cited:
- EP-B1- 0 112 104
- WO-A1-97/47337
- US-A- 4 338 190
- US-A- 4 618 343
- US-A- 5 660 722

## Description

### FIELD OF THE INVENTION

The present invention relates to a peritoneal dialysis instrument, for improving dialysis efficacy in removal of excess liquid and uremic toxin by maintaining polymer osmotic agents in place of glucose, in recirculation line without contact to the outside for therapy of chronic renal failure disease

### BACKGROUND OF THE INVENTION

Peritoneal dialysis has been applied as an effective therapy for renal failure patients. The dialysis is performed so that dialysate is infused into the peritoneal cavity from the dialysate bag, through a catheter, which is implanted in the patient's peritoneal cavity, and the dialysate is stored in the cavity for a certain time, thence the dialysate is drained out through the same catheter. This procedure is repeated a few times a day.

This dialysis has a few advantages over hemodialysis in physiological point of view as it purifies blood continuously through the patients' peritoneum, while hemodialysis uses artificial membrane. Also peritoneal dialysis enables the patients' social activity, so that the dialysis has been widely applied.

In hemodialysis, ultrafiltration is achieved by raising the pressure of blood line over that of the dialysate line. However the same method cannot be applied to peritoneal dialysis, so that an osmotic agent is added into the dialysate so as to raise the osmotio pressure of the dialysate over that of plasma, and the dialysate is infused into the peritoneal cavity so as to contact it to peritoneum for removing excess liquid from the patient's body. For this purpose, glucose has been used as an osmotic agent, however adverse effects such as disfunctioning of peritoneum, due to the absorption of its large quantity into the patient body, are now recognized as a serious problem.

For solving the aforementioned problem, the inventor of the present invention has proposed the instrument and the method by which serum protein such as albumin, globulin and the like which are permeated out through peritoneum into the dialysate, is recovered and refined, then concentrated and reused with dialysate as the most physiological substitutes of glucose.

In these proposed processes, the followings were disclosed;
(A) A method to dissolve the recovered and refined protein in dialysate, after low molecular weight uremic toxin substance, not higher than 30,000 dalton, are removed by the repeated concentration/dilution procedures with semipermeable membrane, and to reuse it as a substitute of glucose. (Japanese Laid Open Patent Application Hei 9-327511)
(B) A method to keep the abovementioned device and the components disinfective. (Japanese Laid Open Patent Application Hei 10-85324)
(C) A method to separate the malignant solute in the solvent and refine the protein by acidifying followed by de-acidifying through water dialysis to deposit at iso-electric pH (Japanese Laid Open Patent Application Hei 9-302388)
   Also for carrying out the invention (C), it was disclosed that the device comprises the followings;
(D) An inflow line having a filter (maximum pore size:100-300nanometer) for preventing bacteria invasion into peritoneal cavity
(E) A two step prefilter (pore size: 200 micron and 5 micron) to remove blood cells, peritoneum mesothelial cells, fibrin and the like suspended in the effluent when it is drained out from peritoneal cavity.

A few attempts have been reported to utilize serum protein in ascites (Hwang, E.R., Richard, D.O. Sherman, A. et al: Dialytic ascites ultra-filtration in refractory ascites: Am..J.Gastroenteral, 77(9) :652-654, 1982 et al)

However they did not refer to removing uremic toxin, because their target was not renal failure patient.

Also a method to add a peritoneum protecting component of molecular weight not higher than 3,000 dalton recovered from peritoneal dialysis effluent into dialysate (Japanese Laid Open Patent Application Hei 8-337590). However the recovery and reuse of the component of the molecular weight higher than 3,000 dalton is not suggested.

When plasma protein, that is permeated out of the patient body, is reused as an osmotic agent in place of glucose, it was needed to satisfy the following cares;
(I) To minimize the contact with atmosphere and foreign matters not to denature the protein
(II) To minimize plugging the semi-permeable membrane on the recirculation line, and to decrease the frequency of exchange.
(III) To prevent the invasion of pathogenic bacteria and endotoxin completely.

For the solution of the aforementioned (I) problem, there may be suggested that a filter is set at the exit of the catheter or, as a further perfect protection, hollow fiber type semi-permeable membrane is set in peritoneal cavity in order to keep the polymer in the peritoneal cavity. However in these cases, complicated preventive means are required to avoid plugging of the membrane, and the exchange of the filter requires skilful cares.

US-A-4338190 discloses the features of the preamble of claim 1.

### DESCRIPTION OF THE INVENTION

The present invention developed a practical method and an instrument for solving the aforementioned problems, by the combination of either ones of the following technologies;
(I) The drained dialysate is warmed up to a preset temperature, thence it is filtered through a prefilter for removing foreign materials, so as to prevent the plugging of the filter.
(II) A semi-permeable membrane (cut-off point: 30,000 dalton) filter is used for removing uremic toxin of low molecular weight and of middle molecular weight.
(III) Supplemental electrolyte solution is supplied through a semi-permeable membrane filter (cut-off point: 5,000 dalton), for preventing the infection and invasion of endotoxin.

Also the present inventor found that by use of the device, dialysate may be drained out of the peritoneal cavity and may be recirculated in a closed line, and a portion of the dialysate may be filtered out through a semipermeable membrane to remove malignant component, and thence a fresh dialysate may be supplemented through a semipermeable membrane and returned into the peritoneal cavity automatically.

According to the present invention, an instrument is provided for continuous recirculation of peritoneal dialysate to infuse and drain out the dialysate automatically through catheters implanted in a peritoneal cavity of a human body, including a prefilter, a primary filter, said primary filter being operable to filter out a portion of the dialysate, a pump operable to lower an outside pressure of said primary filter relative to an inside pressure of said primary filter, a feeding pump operable to supply a fresh dialysate, and a secondary filter located downstream of said feeding pump, said secondary filter being operable to filter out a portion of the fresh dialysate, characterised in that said primary filter comprises a semipermeable membrane having a maximum permeable molecule of up to 30,000 dalton, in that said secondary filter comprises a semipermeable membrane having maximum permeable molecule of up to 5,000 dalton and in that the prefilter, the primary filter and the secondary filter are disposed in series in the recirculation line.

As a favorable embodiment for carrying out the present invention, the following technologies may be adapted;
(1) A bacteria-free filter ( (maximum pore size : 100-300 nanometer) is set up on the peritoneal cavity side of the inflow line's joint.
(2) Dialysate in the peritoneal cavity is recirculated through a perfectly closed and continuously connected and previously disinfected line for keeping the protein not denatured in the automatic dialysate recirculation instrument.
(3) A reverse flow prevention valve (anti-reverse flow valve) is set up on the withdrawal line.
(4) A closed chamber, of which inside cannot directly be contacted with fingers, is set up for disconnection and connection procedure by remote operation from outside, after the infusion of dialysate for the daytime cycle before getting up in the morning.

### BRIEF DESCRIPTION OF THE DRAWINGS

### Figure 1

Peritoneal Dialysate Recirculation Circuit (Night time state: Peritoneal dialysate recirculation instrument is connected with the patient's outflow and inflow catheters respectively and the dialysate is recirculated)

### Figure 2

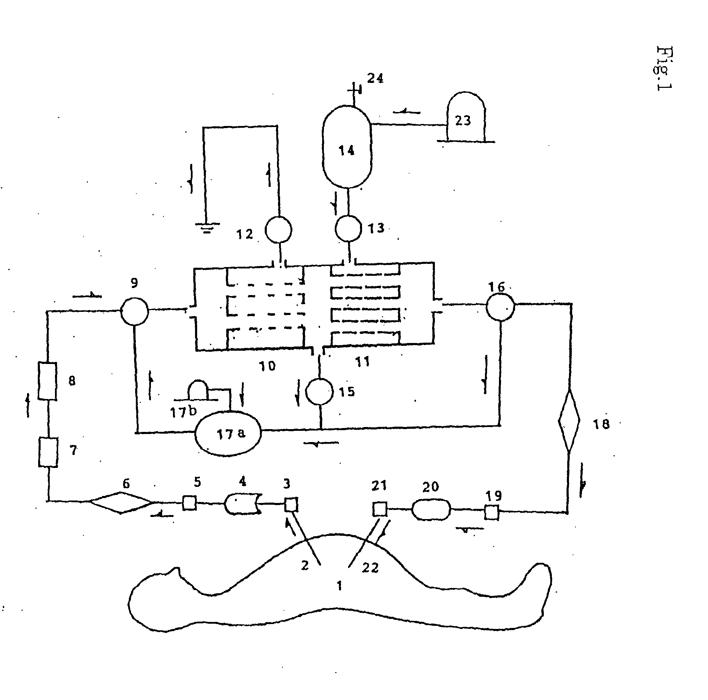

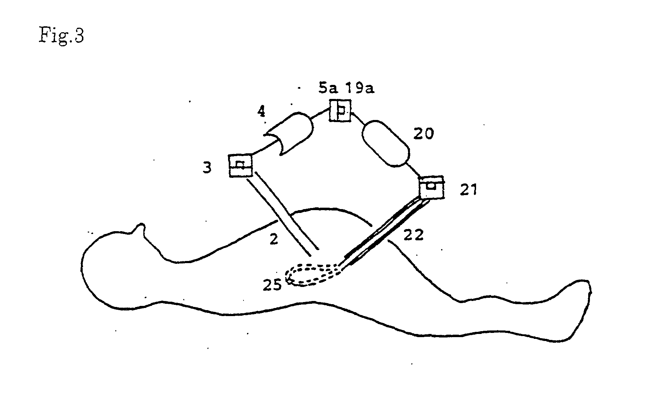

Exchanging method of outflow line joint and inflow line joint.
(a) disconnecting operation of the joint, which has been directly connected in daytime (Figure3), and rotation operation of the parts.
(b) rotating operation of the disconnected part so as to face the part of peritoneal cavity side and the part of recirculation instrument side, and connecting operation of the parts so as to make ready for night time recirculation (Figure 1).

### Figure 3

O - shaped circuit of the catheter at extracorporeal side ( day time state when the patient leaves away from the recirculation instrument for daily life) and hollow fibers in the peritoneal cavity.

### Explanation of the Marks

- 1.: Peritoneal Cavity
- 2.: Outflow Catheter
- 3.: Joint
- 4.: Anti-Reverse Flow Valve
- 5.: Outflow Line Joint
5a Peritoneal Cavity Side Part of Outflow Line Joint
5b Recirculation Instrument Side Part of Outflow Line Joint
- 6.: Heater
- 7.: Prefilter
- 8.: Bacteria-free Filter
- 9.: Pump
- 10.: Primary Filter
- 11.: Secondary Filter
- 12.: Pump
- 13.: Pump
- 14.: Supplemental Solution
- 15.: Pump
- 16.: Pump
17a Container
17b Reservoir of Osmotic Agents
- 18: Warmer
- 19: Inflow Line Joint
- 19a: Peritoneal Cavity Side Part of Inflow Line Joint
- 19b: Recirculation Instrument Side Part of Inflow Line Joint
- 20: Bacteria -free Filter
- 21: Joint
- 22: Inflow Catheter
- 23: Reverse Osmosis Membrane Water
- 24: Inlet Valve of Chemicals
- 25: Loope-shape Hollow Fibers

### MOST PREFERRABLE EMBODIMENT FOR CARRYING OUT THE INVENTION

The present invention will be explained by Figure 1.
Figure 1 illustrates outflow catheter 2 and inflow catheter 22 in peritoneal cavity 1.

It happens to be observed often that when liquor is recirculated from an inflow entrance to outflow exit at a consistent rate, a localized flow, so-called channeling is formed in peritoneal cavity; then a portion of the liquor tends to stay at "dead spaces". For solving this problem, a certain number of loope-shaped porous hollow fibers 25 are fixed at the end of inflow catheter, so that the dialysate may flow throughout in the cavity as illustrated in Figure 3. In place of outflow catheter 2, an outer lumen of a co-centric double lumen catheter may take place as well.

Outflow catheter 2 comprises joint 3, anti-reverse flow valve 4, outflow joint 5 and connected with heater 6 and prefilter 7 in a series.

Outflow joint 5 comprises peritoneal side part 5a and instrument side part 5b as illustrated in Figure 3. During recirculation time at night, the parts 5a and 5b are connected together. The joint 5 has the structure of male/female, which are directly adaptable to each counter parts of inflow joint 19 as mentioned later. During the time when dialysate is not recirculated but stored in peritoneal cavity in day time, the joint part 5a is connected with the joint part 19a, and the joint part 5b is connected with the joint part 19b, forming the circuit illustrated in Figure 3.

The dialysis effluent that is drained out of the patient's peritoneal cavity contains peritoneum mesothelium cells, leucocyte cells, deposited fibrin, and the like. These foreign particles may be separated with prefilter 7 from the filtrate.

Fibrinogen in the dialysate effluent tends to be deposited out as fibrin after prefiltration, and plugs the filter. The fact has been experienced often when plasma and humor is filtered. For preventing the plugging problems, it is desirable to warm up the effluent up to 55-60°C through heater before prefiltration.

After the dialysate is passed through a bacteria-free filter 8, it is made to flow by pump 9 to the first filter 10 and thence the second filter 11. The first filter has semipermeable membrane of maximum permeable molecule, greater than that of 2.-microglobulin, for example, 30,000 dalton. By filtering out a portion of the dialysate through this filter, middle molecule malignant components, such as 2-microglobulin of the molecular weight 11,800 dalton, may be removed.

After filtering through the first filter, the partially filtered dialysate is supplemented with supplemental electrolyte solution. The supplement solution is added through the second filter of which the semipermeable membrane does not pass endotoxin. The second filter has the semipermeable membrane of maximum permeable molecule, 5,000 dalton, so that it can prevent invasion of bacteria and endotoxin.

Endotoxin is a lipopolysaccharides, of which the largest ones have molecular weight of a few hundred thousands dalton. The smallest lipopolyssaccharides have molecular weight, 6,000-8,000 dalton. On the other hand, supplemental chemicals and additives are low molecules, such as 1,000 dalton, so that it may be added through this semipermeable membrane of the secondary filter.

Due to the reduced pressure in the outside of the first filter 10 by pump 12, dialysate in the first filter 10 is suctioned out. The supplement solution in the second filter is pressed by pump 13 to feed in through the second filter 11. The filtration in both filters is accelerated by these pumps.

The supplemental solution is stored in a container 14, and it is sent to the second filter by pump 13. Amino acids, fatty acids, glucose, peptides or any mixture of these are added into the supplemental solution through a line which is connected with a valve 24 which is equipped in the container.

The above-mentioned supplemental solution may be
(a) commercially available infusion solution or peritoneal dialysate which is sterilized and packed in a container 14, or
(b) hemodialysis concentrate or dry chemicals for hemodialysis, which is diluted or dissolved with, reverse osmosis water.

After partial filtration out in the first filter and supplementation at the second filter, the dialysate is made to flow by pump 16 through a warmer 18, where it is warmed up to a standard corporeal temperature, then infused through inflow joint 19, bacteria -free filter 20 and joint 21, into peritoneal cavity

On the by-pass line 15-17a-9, a container 17a is set up, where a portion of polymer components, which is stored in peritoneal cavity in day time, may be stored. By pump 15, the solution can be circulated through the line so as to repeat concentration/dilution procedures. For the container 17a, cooling or freezing unit may be equipped.

One of the present invention's aims is reuse of recovered plasma protein permeated from patient body through peritoneum into the dialysate. However in the case of the recovered protein is not enough to required ultrafiltration, other osmotic agents may be supplemented. Such a supplemental agents may be high or low molecular weight substances.
High molecular ones may be oligosaccharides, and low molecular weight substances may be glucose or amino acids. Even when substances, of which daily dose is restricted, are used, usage is within the tolerable quantity, those osmotic agents may be used so that the required osmotic pressure can be obtained. Low molecular weight agents are added from a supplemental reservoir 14, and high molecular weight agents are supplied from an osmotic agent reservoir 17b into a container 17a, where the additives are mixed with the dialysate.

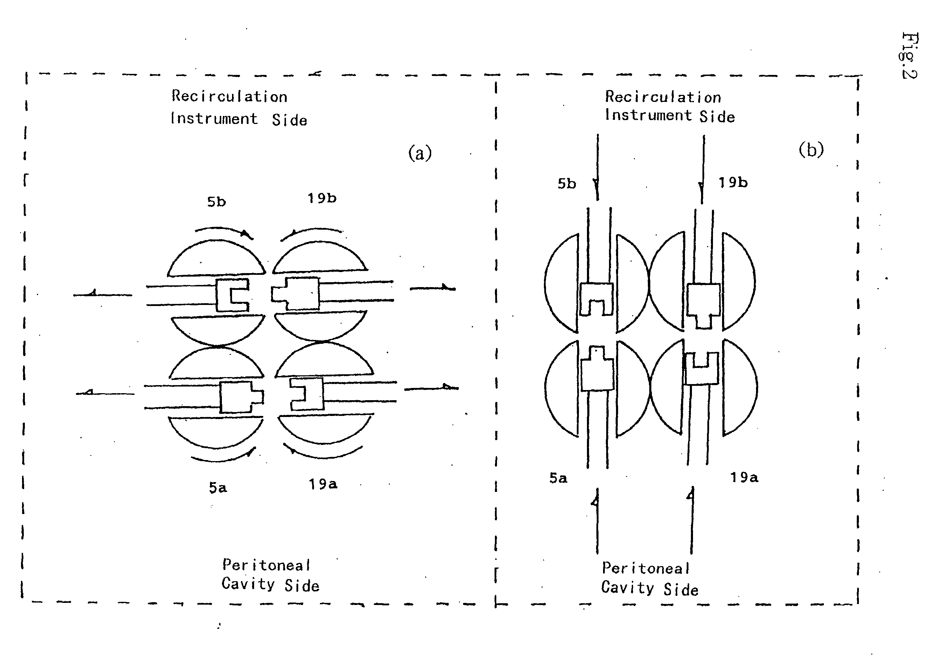

The recirculation instrument is connected with peritoneal catheters at night so as to achieve peritoneal dialysate recirculation automatically. However in day time, joint 5 and joint 19 are disconnected from the recirculation instrument, and form a day time circuit. For such a disconnection and connection operation, each joint comprises part a and part b as illustrated in Figure 2. That is, joint 5 consists of 5a (male) and 5b (female), joint 19 consists of 19a (female) and 19b (male). When 5a and 5b is disconnected, as well as 19a and 19b, 5a and 19a can be connected, as well as 5b and 19b as illustrated in Figure 3. According to the present invention, outflow joint 5 and inflow joint 19 are set up adjacently in a case and manipulated from outside of the case free from finger touch.

By use of the recirculation instrument according to the present invention, extraperitoneal recirculation procedures may be achieved continuously and automatically in the following way. First of all, before sleeping, parts 5a and 19a and parts 5b and 19b, which has been connected respectively in day time, are disconnected. Thence each part is rotated by 90 degree to the direction along the arrows as illustrated in Figure 2. Then 5a and 5b is connected as well as 19a and 19b to form a recirculating circuit as illustrated in Figure 1.

When the circuit line is set up, recirculation is started. After concentrating the drained dialysate and removing uremic toxin in the first filter, a portion of the concentrate is stored in a container 17a.
The remaining concentrate is added fresh electrolyte solution through the second filter 11, then infused into peritoneal cavity. If needed, concentrating/diluting procedures are repeated a few times through a circulation circuit (16―17a―9). In some case, electrolyte solution is added amino acids, glucose, fatty acids, or peptides etc.

Not only sodium caprilate and N-acetyltryptophan as stabilizers to prevent the recycled protein denatured, but also acids, alkali, anti-oxidants, such as, glutathione, vitamin C, vitamin E and reductants are added to electrolyte solution, so as to release urea, bilirubin, and S-S bonded chemicals that are attached to cysteine, 34^{th} amino acid from N-terminal of albumin. Through making albumin as active as that of healthy persons by the abovementioned way before infusing into peritoneal cavity, it may improve the therapy effect.

Thus, the dialysate in peritoneal cavity is consistently drained out, and is substituted partly with fresh electrolyte solution on the way of recirculation at night when the patient sleeps.

Before getting up in the morning, all or almost all of the dialysate in peritoneal cavity is drained out, and the drain is repeatedly concentrated and diluted, thence the aforementioned chemicals are added, and infused into the peritoneal cavity. The joints 5 and 19 are disconnected to form a circuit as illustrated in Figure 3 by connecting the part 5 with the part 19 directly. Briefly, as in Figure 3, on the catheter side, "O"shape circuit is formed. In the catheter side, the part 19a of a bacteria-free filter entrance 20 on inflow line and the part 5a of an anti- reverse flow-valve exit 4 on outflow line are connected. On the recirculation instrument side, the counter parts 5b and 19b are connected.

The above-mentioned operation can be manipulated in a separate case to which fingers cannot touch, and through which continuous recirculation of the dialysate can be performed.

By use of the instrument according to the present invention, continuous recirculation can be achieved simultaneously to partial substitution of the dialysate.

### INDUSTRIAL APPLICABILITY

By the instrument according to the present invention, reusing the permeated out protein into the peritoneal dialysate safely, and continuous recirculation of the dialysate can be achieved in the simplest way. Briefly, every day, dialysate is drained out and infused through semipermeable membrane, solution flows through completely closed circuit line so as to minimize risk of infection.

By the instrument according to the present invention, continuous recirculation of the dialysate can be achieved simultaneously a partial substitution, In this way continuous draining of the dialysate out of peritoneal cavity, and partial substitution of the dialysate with fresh electrolyte solution during sleeping at night. After getting up in the morning, the patient can leave away from the instrument after disconnection operation and enjoy daily life in daytime.

It has been said that for improvement in dialysance of peritoneal dialysis, increasing the number of dialysis cycles per day is effective, however too many cycles of peritoneal dialysis increases time in vacancy of peritoneal cavity. For dissolving this problem, tidal type recirculation which leaves a portion of liquid in the peritoneal cavity. But it can not improve dialysance significantly.

The present invention, in contrast, can improve the dialysance, as the dialysate recirculates without vacancy time in peritoneal cavity. Another recirculating method, that the dialysate is refined by extraperitoneal dialysis by use of artificial dialyser and extracoporial dialysate, can improve the dialysance, but it requires a large volume of dialysate. The present invention provides much more economical dialysis by a partial substitution of recirculated dialysate. This advantage is also valid in the case of no polymer component is contained and recycled.

For reducing the therapy cost, instead of large volume of dialysate delivered, on site preparation of dialysate by diluting the dialysate concentrate or dissolving dry chemicals is very effective. The water preparation device for the dissolution and dilution by reverse osmosis membrane may be equipped in the instrument according to the present invention, so as to provide safe and low cost dialysate.

Infection can be prevented by use of previously connected, packed and sterilized extracorporeal recirculation line. Also the infection rate at periodical exchange can be extremely reduced by the way that outflow and inflow connection parts are fixed adjacent in a closed case as illustrated in Figure 2, as the connection parts can be disconnected and exchanged by outside manipulation free from finger touch to the parts.

## Claims

1. An instrument for continuous recirculation of peritoneal dialysate to infuse and drain out the dialysate automatically through catheters implanted in a peritoneal cavity of a human body, including a prefilter (7), a primary filter (10) said primary filter being operable to filter out a portion of the dialysate, a pump (12) operable to lower an outside pressure of said primary filter (10) relative to an inside pressure of said primary filter, a feeding pump (13) operable to supply a fresh dialysate, and a secondary filter (11) located downstream of said feeding pump (13), said secondary filter being operable to filter out a portion of the fresh dialysate, **characterised in that** said primary filter (10) comprises a semipermeable membrane having a maximum permeable molecule of up to 30,000 dalton, **in that** said secondary filter (11) comprises a semipermeable membrane having maximum permeable molecule of up to 5,000 dalton and **in that** the prefilter (7), the primary filter (10) and the secondary filter (11) are disposed in series in the recirculation line.

2. The instrument for continuous recirculation of peritoneal dialysate according to claim 1, wherein a supplemental liquor line provides communication between said feeding pump (13) and said secondary filter (11).

3. The instrument for continuous recirculation of peritoneal dialysate according to claim 1 or 2, including an instrument side part of an outflow joint (17) for connection to a peritoneal side part of the outflow joint on a peritoneal catheter and including an instrument side part of an inflow joint (19) for connection to a peritoneal side part of the inflow joint on a peritoneal catheter; these instrument side parts having a structure so as to be directly connectable to each other; the instrument also providing an isolation arrangement in which the aforementioned joints may be fixed adjacent each other so that the instrument side joint parts (5a,5b) and the peritoneal side joint parts (19a,19b) may be disconnected from and connected to each other by remote handling free, from finger touch.

## Patentansprüche

1. Gerät zur kontinuierlichen Rezirkulation von Peritoneal-Dialysat zur automatischen Infusion oder Drainage des Dialysats durch Katheter, die in eine Peritoneal-Höhle eines menschlichen Körpers implantiert sind, mit einem Vorfilter (7), einem Hauptfilter (10), wobei der Hauptfilter dahingehend funktionsfähig ist, einen Teil des Dialysats herauszufiltern, einer Pumpe (12), die dahingehend funktionsfähig ist, einen Außendruck des Hauptfilters (10) im Verhältnis zu einem Innendruck des Hauptfilters herabzusetzen, einer Zuführpumpe (13), die dahingehend funktionsfähig ist, ein frisches Dialysat zu liefern, und einem Sekundärfilter (11), der stromabwärts der Zuführpumpe (13) angeordnet ist, wobei der Sekundärfilter dahingehend funktionsfähig ist, einen Teil des frischen Dialysats herauszufiltern, **dadurch gekennzeichnet, dass** der Hauptfilter (10) eine semipermeable Membran mit einem maximal hindurchtretenden Molekül von bis zu 30.000 Dalton umfasst, dass der Sekundärfilter (11) eine semipermeable Membran mit einem maximal hindurchtretenden Molekül von bis zu 5.000 Dalton umfasst, und dass der Vorfilter (7), der Hauptfilter (10) und der Sekundärfilter (11) in der Rezirkulationsleitung in Reihe angeordnet sind.

2. Gerät zur kontinuierlichen Rezirkulation von Peritoneal-Dialysat nach Anspruch 1, bei dem eine zusätzliche Liquor-Leitung eine Verbindung zwischen der Zuführpumpe (13) und dem Sekundärfilter (11) bereitstellt.

3. Gerät zur kontinuierlichen Rezirkulation von Peritoneal-Dialysat nach Anspruch 1 oder 2, mit einem geräteseitigen Teil eines Auslassanschlusses (5) zum Verbinden mit einem peritonealseitigen Teil des Auslassanschlusses an einem Peritoneal-Katheter und mit einem geräteseitigen Teil eines Einlassanschlusses (19) zum Verbinden mit einem peritonealseitigen Teil des Einlassanschlusses an einem Peritoneal-Katheter, wobei diese geräteseitigen Teile einen Aufbau derart aufweisen, dass sie direkt miteinander verbindbar sind, wobei das Gerät ferner eine Isolierungsanordnung bereitstellt, in der die vorgenannten Anschlüsse nebeneinander befestigt werden können, so dass die geräteseitigen Anschlussteile (5b, 19b) und die peritonealseitigen Anschlussteile (5a, 19a) durch Fernhandhabung, ohne Fingerberührung, voneinander getrennt und miteinander verbunden werden können.

## Revendications

1. Appareil de recirculation en continu d'un liquide de dialyse péritonéale, pour infuser et évacuer le liquide de dialyse automatiquement par l'intermédiaire de cathéters implantés dans une cavité péritonéale d'un corps humain, appareil comprenant un filtre préliminaire (7), un filtre primaire (10) permettant de séparer par filtration une partie du liquide de dialyse, une pompe (12) permettant d'abaisser la pression du côté externe dudit filtre primaire (10) par rapport à la pression du côté interne dudit filtre primaire, une pompe d'alimentation (13) permettant de fournir un liquide de dialyse frais, et un filtre secondaire (11) situé en aval de ladite pompe d'alimentation (13) et permettant de séparer par filtration une partie du liquide de dialyse frais, appareil **caractérisé en ce que** ledit filtre primaire (10) comprend une membrane semi-perméable ayant un seuil de coupure de 30 000 daltons, **en ce que** ledit filtre secondaire (11) comprend une membrane semi-perméable ayant un seuil de coupure de 5000 daltons, et **en ce que** le filtre préliminaire (7), le filtre primaire (10) et le filtre secondaire (11) sont disposés en série dans le circuit de recirculation.

2. Appareil de recirculation en continu d'un liquide de dialyse péritonéale selon la revendication 1, dans lequel une ligne de liqueur supplémentaire établit une communication entre ladite pompe d'alimentation (13) et ledit filtre secondaire (11).

3. Appareil de recirculation en continu d'un liquide de dialyse péritonéale, selon la revendication 1 ou 2, qui comprend une partie côté appareil d'un raccord (5) de courant de sortie, destinée à être reliée à une partie côté péritonéal du raccord de courant de sortie, située sur un cathéter péritonéal, et qui comprend une partie côté appareil d'un raccord (19) de courant d'entrée, destinée à être reliée à une partie côté péritonéal du raccord de courant d'entrée, située sur un cathéter péritonéal, ces parties côté appareil ayant une structure telle qu'elles peuvent être raccordées directement l'une à l'autre, l'appareil fournissant également un dispositif d'isolement dans lequel les raccords mentionnés précédemment peuvent être fixés en étant adjacents l'un à l'autre, de sorte que les parties des raccords côté appareil (5b, 19b) et les parties des raccords côté péritonéal (5a, 19a) peuvent être déconnectées les unes des autres et raccordées les unes aux autres par manipulation à distance, sans contact avec les doigts.
